Europäisches Patentamt

(19) European Patent Office

Office européen des brevets

(11) Veröffentlichungsnummer: **0 394 712**
**A2**

## (12) EUROPÄISCHE PATENTANMELDUNG

(21) Anmeldenummer: **90106331.3**

(22) Anmeldetag: **03.04.90**

(51) Int. Cl.⁵: **G01N 15/02, F17C 13/04**

(30) Priorität: **22.04.89 DE 3913256**

(43) Veröffentlichungstag der Anmeldung:
**31.10.90 Patentblatt 90/44**

(84) Benannte Vertragsstaaten:
**AT DE ES FR NL**

(71) Anmelder: **Linde Aktiengesellschaft**
**Abraham-Lincoln-Strasse 21**
**D-6200 Wiesbaden(DE)**

(72) Erfinder: **El Shamaa, Fouad, Chem.-Ing.**
**Starenweg 3**
**D-8033 Krailling(DE)**
Erfinder: **Bomhard, Klaus, Dipl.-Phys.**
**Mainberger Strasse 28**
**D-8000 München 70(DE)**

(74) Vertreter: **Schaefer, Gerhard, Dr.**
**Linde Aktiengesellschaft Zentrale**
**Patentabteilung**
**D-8023 Höllriegelskreuth(DE)**

(54) Einrichtung zur Partikelmessung in Gasen.

㊄ Es handelt sich um eine Einrichtung zur Partikelmessung in Gasen. Dazu sind bekanntermaßen ein Druckreduziersystem und eine Partikelmesseinrichtung notwendig. Gängige Druckreduziersysteme, wie beispielsweise Druckminderer, besitzen den Nachteil, daß sie gegeneinander bewegliche, Partikel erzeugende oder absorbierende Bauteile enthalten. Deshalb wird vorgeschlagen, als Druckreduziersystem eine überkritische Düse in der Zuleitung zur Partikelmeßeinrichtung anzuordnen.

EP 0 394 712 A2

## Einrichtung zur Partikelmessung in Gasen

Die Erfindung betrifft eine Einrichtung zur Partikelmessung in Gasen mit einem Druckreduziersystem und und einer nachfolgenden Partikelmeßeinrichtung.

Insbesondere für Gasanwendungen, bei denen nur eine geringe Anzahl von Partikeln im Gas mitgeführt werden darf, ist eine Messung und Spezifizierung der mitgeführten Partikel notwendig.

Bei der Spezifizierung eines Gases mit seinem jeweiligen Speichersystem, z.B. einer Gasflasche oder einem Flüsiggastank, besteht das Problem, daß der mit einem Partikelmeßeinrichtung gemessene Wert oft nicht zutrifft, da durch die dem Partikelmeßeinrichtung vorgeschalteten Armaturen zur Druckminderung eine Verfälschung der ursprünglichen Partikelzahl auftritt. Bisher sind nämlich für diese Aufgabe Einrichtungen bekannt, bei denen zur Druckminderung vor dem Partikelmeßeinrichtung gängige Elemente, wie Druckminderer und Dosierventile, eingesetzt werden, wobei jedoch aufgrund der in diesen Elementen vorhandenen, gegeneinander bewegten mechanischen Bauelemente und Dichtungstoffe besagte Verfälschung verursacht wird, nämlich Partikel generiert oder absorbiert werden. Dieser Effekt ist insbesondere bei hohen Eingangsdrücken wesentlich.

Die Aufgabe der vorliegenden Erfindung besteht deshalb darin, eine Einrichtung zur Partikelmessung anzugeben, die ein weitgehend unverfälschtes Meßergebnis für die Partikelanzahl insbesondere auch bei hohen Eingangsdrücken liefert.

Diese Aufgabe wird erfindungsgemäß dadurch gelöst, daß als Druckreduziersystem eine beispielsweise aus der Trägergasregelung bei Gas-Chromatographen bekannte, überkritische Düse in der Zuleitung zum Partikelmeßeinrichtung angeordnet ist.

Diese Problemlösung ist ebenso einfach wie vorteilhaft. Eine überkritische Düse besteht praktisch im wesentlichen aus einer Scheibe mit einer im Vergleich zu ihrer Fläche kleinen Öffnung. Eine Düse, deren Lochdurchmesser so klein gewählt wird, daß bei strömendem Gas das kritische Druckverhältnis ( $p_2/p_1$ )$_{krit.}$ unterschritten wird ( $p_2$: Gasdruck nach der Düse, $p_1$: Gasdruck vor der Düse ), liefert bei konstanter Temperatur einen konstanten Volumenstrom, der nicht vom Druck $p_2$ hinter der Düse abhängt. Die dadurch bewirkte Strömungsregelung und Druckeinstellung wird also ohne die geeignete Einstellung gegeneinander bewegbarer mechanischer Bauteile bewirkt, wie dies bei den gängigen Druckeinstellelementen der Fall ist. Deshalb treten mit der Anwendung einer überkritischen Düse als Druckreduziersystem keinerlei zusätzliche Partikel auf oder werden absorbiert, und man erhält durch die anschließende Partikelmessung ein unverfälschtes Ergebnis, das eine genaue Spezifizierung des jeweiligen Ausgangsgases zusammen mit der zugehörigen Speichereinheit ermöglicht.

Um temperaturbedingte Veränderungen des Meßergebnisses auszuschließen, die aufgrund der Drucksenkung im Meßgas auftreten können, ist es vorteilhaft, zwischen überkritischer Düse und Partikelmeßeinrichtung eine Temperaturausgleichsvorrichtung vorzusehen. Als Temperaturausgleichsvorrichtung kann beispielsweise mit Vorteil ein Wasserbad vorgesehen werden, durch das die Zuleitung zur Partikelmeßeinrichtung zu führen ist.

In einer günstigen Ausgestaltung der erfindungsgemäßen Einrichtung wird die überkritische Düse in die Kupplung zweier Leitungsstücke, die zur Zuleitung zum Partikelmeßeinrichtung gehören, installiert. Dabei ist es besonders vorteilhaft, wenn die Installation der überkritischen Düse an der Kopplungsstelle der Leitungsstücke mit Hilfe einer dort einsetzbaren Fassung erfolgt, in die eine Stützblende eingelegt ist, auf der dann die überkritische Düse aufliegt.

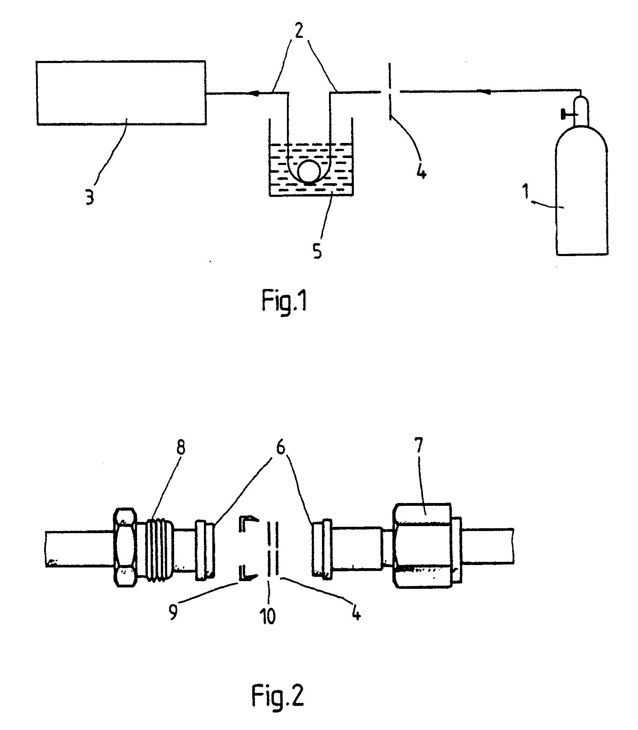

Die erfindungsgemäße Einrichtung soll im folgenden anhand der Figuren 1 und 2 beispielhaft näher erläutert werden.

Es zeigt

Figur 1 den schematischen Aufbau der erfindungsgemäßen Einrichtung,

Figur 2 eine Ausgestaltung der Installation einer überkritischen Düse in einer erfindungsgemäßen Einrichtung.

In Figur 1 ist eine Gasflasche 1 dargestellt, die über eine Zuleitung 2 mit einer Partikelmeßeinrichtung 3 verbunden ist. In der Zuleitung 2 folgt stromabwärts von der Gasflasche 1 eine überkritische Düse 4, worauf folgend die Zuleitung 2 durch ein Wasserbad 5 geführt ist.

Die zu spezifizierende, unter Druck $p_1$ stehende Gasprobe tritt mit einer im wesentlichen von $p_1$ und vom gewählten Durchmesser der überkritischen Düse abhängigen Strömung aus der Düse aus. Dabei kann prinzipiell mit jedem Eingangsdruck gearbeitet werden, solange das kritische Druckverhältnis unterschritten wird, wobei die Vorteile der erfindungsgemäßen Einrichtung insbesondere bei höheren Drücken ($p_1$ größer 10 bar) zum Tragen kommen, da in diesen Druckbereichen aufgrund der eingangs genannten Nachteile nicht mehr mit konventionellen Druckreduziersystemen gearbeitet werden kann. Eingangsdrücke bis über 300 bar, wie sie aus bekannten Gasspeichersystemen geliefert werden können, können ebenso verarbeitet werden, wobei in der Regel eine Druckreduzierung auf Umgebungsdruck erfolgt, da dieser von den Partikelmeßgeräten verarbeitet werden kann. Die Düsenöffnung ist dabei abhängig vom Molekulargewicht des Meßgases, dem gewünschten Volumenstrom und dem Eingangsdruck. Im allgemeinen treten dabei Öffnungsdurchmesser zwischen 20 und 300 Mikrometern auf.

Die Figur 2 zeigt eine günstige Installation einer überkritischen Düse in die Zuleitung 2. Dazu muß in der Zuleitung 2 eine Kopplungsstelle vorgesehen sein. An dieser Kopplungsstelle sind Stirnflächen 6 durch eine Verbindungsschraube 7 mit zugehörigem Gegengewinde 8 aufeinander preßbar. Zur Installation der überkritischen Düse ist eine auf die Stirnflächen 6 passende, mit umgebördeltem Rand und freiem Innendurchmesser ausgestattete Fassung 9 vorgesehen, in die neben der überkritischen Düse zusätzlich auf der Niederdruckseite eine Stützblende 10 eingelegt wird. Diese Einheit aus Fassung, Stützblende und überkritischer Düse ist in ihrer Gesamtheit auf eine Stirnfläche 6 aufsetzbar und die Kopplungsstelle auch nach Anbringen dieser Einheit problemlos verschraubbar. Damit ist eine günstige Installationsmöglichkeit der erfindungsgemäß erforderlichen überkritischen Düse gegeben.

## Ansprüche

1. Einrichtung zur Partikelmessung in Gasen mit einem Druckreduziersystem und einer nachfolgenden Partikelmeßeinrichtung, dadurch gekennzeichnet, daß als Druckreduziersystem eine überkritische Düse in der Zuleitung zur Partikelmeßeinrichtung angeordnet ist.

2. Einrichtung nach Anspruch 1, dadurch gekennzeichnet, daß zwischen überkritischer Düse und Partikelmeßeinrichtung eine Temperaturausgleichs- vorrichtung für das Meßgas vorgesehen ist.

3. Einrichtung nach Anspruch 2, dadurch gekennzeichnet, daß die Temperaturausgleichsvorrichtung ein Wasserbad, durch das die Zuleitung zur Partikelmeßeinrichtung geführt wird, ist.

4. Einrichtung nach einem der Ansprüche 1 bis 3, dadurch gekennzeichnet, daß die überkritische Düse in die Kupplung zweier Leitungsstücke, die die Zuleitung zur Partikelmeßeinrichtung bilden, installiert ist.

5. Einrichtung nach Anspruch 4, dadurch gekennzeichnet, daß die Installation der überkritischen Düse an der Kupplungsstelle zweier Leitungsstücke mit Hilfe einer dort eisetzbaren Fassung erfolgt, in die eine Stützblende eingelegt ist und auf der die überkritische Düse aufliegt.

Fig.1

Fig.2